# EUROPEAN PATENT SPECIFICATION

(11) **EP 0 981 746 B1**
(45) Date of publication and mention of the grant of the patent: **03.09.2003**
(21) Application number: 98908381.1
(22) Date of filing: 27.02.1998
(51) Int. Cl.: G01N 33/53, G01N 33/68

(54) **IMMUNOASSAY AND KIT FOR IgA ANTIBODIES SPECIFIC FOR ACETALDEHYDE ADDUCTS**
IMMUNOASSAY UND KIT FÜR IgA ANTIKÖRPER, SPEZIFISCH FÜR ACETALDEHYD-ADDUKTE
DOSAGE IMMUNOLOGIQUE ET KIT POUR ANTICORPS IgA SPECIFIQUES D'ADDUITS D'ACETALDEHYDE

(30) Priority: 03.03.1997 SE 9700745
(43) Date of publication of application: 01.03.2000
(73) Proprietor: AXIS-SHIELD ASA, 0581 Oslo 5 (NO)
(72) Inventor: SILLANAUKEE, Pekka, S-756 53 Uppsala (SE); HURME, Liisa, S-753 25 Uppsala (SE); WORRALL, Simon, Sinnamon Park, QLD 4073 (AU)
(74) Representative: Golding, Louise Ann
(86) International application number: SE9800351
(87) International publication number: WO98039652

(56) References cited:
- US-A- 4 647 654
- SIMON WORRALL et al., "Relationship Between Alcohol Intake and Immunoglobulin A Immunoreactivity with Acetaldehyde-Modified Bovine Serum Albumin", ALCOHOLISM: CLINICAL AND EXPERIMENTAL RESEARCH, Volume 20, August 1996, pages 836-840.
- PEKKA SILLANAUKEE et al., "Structural Characterisation of Acetaldehyde Adducts Formed by a Synthetic Peptide Mimicking the N-Terminus of the Hemoglobin beta-Chain Under Reducing and Nonreducing Conditions", EUR. J. BIOCHEM., Volume 240, 1996, pages 30-36.

## Description

The present invention concerns an improved immunoassay method for the measurement of IgA antibodies directed against acetaldehyde adducts of proteins. This type of assays is useful for the diagnosis of high alcohol intake including both non-alcoholic heavy drinkers and alcoholics.

Recently, the presence of antibodies reactive with acetaldehyde modified protein epitopes in the plasma of alcoholics has been linked to alcohol abuse. However, these antibodies have also been observed in patients with non-alcoholic liver disease and are as such considered unspecific for high alcohol intake (Niemela et al., Hepatology 7 (1987) 1210-1214; Hoerner et al., Hepatology 8 (1988) 569-574; Worrall et al., Alcohol Alcoholism 25 (1990) 509-511). IgA class antibodies against acetaldehyde modified proteins have been shown to be elevated in alcoholics (Worrall et al., Eur. L. Clin. Invest. 21 (1991) 90-95) and heavy drinkers (Worrall et al., Alcoholism: Clin. Exp. Res. 20(1996) 836-840) as compared to social drinkers, and in contrast to total Ig reactivity, IgA reactivity was not elevated in non-alcohol related liver disease.

The methods used for determining IgA antibody titres against procein acetaldehyde adducts have utilized immunoassays involving formation and detection/determination of the complex:
anti-IgA -- sample IgA -- adduct
Adduct stands for an in vitro prepared acetaldehyde adduct of a protein and it functions as an antigen in the assay. The protein used for synthesis of the adduct has been bovine serum albumin or bovine hemoglobin. Sample IgA is self-explanatory. Anti-IgA stands for added antibodies specific for IgA. It has been found that the correlation to high alcohol intake will be improved in case one uses adduct specific reactivity (ASR) which is obtained by subtracting the IgA titre obtained with the unmodified antigen from the IgA titre obtained with the corresponding adduct (Worrall et al., Alcoholism: Clin. Exp. Res. 20(1996) 836-840).

Acetaldehyde, the primary metabolite of ethanol, has been shown to accumulate in the blood and liver following chronic ethanol intake (Nuutinen et al., Alcoholism: Clin. Exp. Res. 7 (1983) 163-168 and Nuutinen et al., Eur. J. Clin. Invest. 14 (1984) 306). Multiple forms of acetaldehyde adducts of various stability are formed in vitro with proteins (Donohue et al., (Arch. Biochem. Biophys 220 (1983) 239-246), lipids, nucleic acids and carbohydrates (Nicholls et al., In. J. Biochem. 24 (1992) 1899-1906) are formed in vitro. Hemoglobin adducts formed in vitro have been detected by Sillanaukee et al (Alcohol. Clin. Exp. Res. 14 (1990) 842-846), Hazelett et al (Alcohol. Clin. Exp. Res. 17 (1992) 1107-1111) and Itälä et al (Anal. Biochem. 224 (1995) 323-329), and in vivo by Sillanaukee et al (Alcohol Alcoholism. 26 (1991) 5-6); SillanauKee et al (Alcohol. 8 (1991) 377-381) and Sillanaukee et al J. Lab. Clin. Mod. 120 (1992) 42-47).

Serum/plasma, levels of acetaldehyde protein adducts have been measured by the use of ELISA methods employing polyclonal antibodies (Lin et al., Alcohol. Clin. Exp. Res. 3 (1993) 669-674; Sillanaukee et al J. Lab. Clin. Med. 120 (1992) 42-47; Niemelä and Israel, Lab. Invest. 67 (19992) 246-252; Niemelä et al., Alcohol. Clin. Exp. Res. 14 (1990) 838-841; and Lin et al., Alcohol. Clin. Exp. Res. 14 (1990) 438-443) produced against acetaldehyde adducts of hemoglobin or against synthetic peptides designed to mimic the sequences of the hemoglobin β-chain, which are theoretically reactive with acetaldehyde (Lin et al., Alcohol. Clin. Exp. Res. 3 (1993) 669-674). Monoclonal antibodies against synthetic peptide adducts have been described (Klassen et al., Alcohol. Clin. Exp. Res. 18 (1994) 164-171; Thiele et al., Biochem. Pharmacol. 48 (1994) 183-189; and Lin et al., Alcohol. Clin. Exp. Res. 19 (1995) 314-319). The use of monoclonal antibodies for measuring clinical relevant serum/plasma levels of acetaldehyde adducts has so far not been reported.

Acetaldehyde may react with primary and secondary amino groups in proteins, i.e. lysine and the N-terminal amino group of proteins. The initial product is a Schiff base that may be stabilised by further reaction to an ethylated amine, or, in N-terminal amino groups, also to a 2-methyl-imidazolidine-4-one. Adduct formation with amino terminal peptides derived from the hemoglobin β-chain (VHLTPEK and VHLTPEC) under various conditions have been studied (Lin et al., Alcohol. Clin. Exp. Res. 19 (1995) 314-319), and Sillanaukee et al., Eur. J. Biochem. 240 (1996) 30-36).

It remains to be clarified which protein part sequences are important for forming acetaldehyde adducis in vivo, and which adduct structures are immunogenic in the sense that they create an elevated IgA reactivity due to high alcohol intake.

### Objectives of the invention

A first objective of the invention is to provide synthetic well-defined and simplified acetaldehyde adduces that can be ussd for- measuring human IgA antibodies specific for acetaldehyde protein adducts produced in vivo as a consequence of high alcohol intake in alcoholics and in heavy drinkers.

A second objective is to minimize sample IgA reactivity to the unmodified antigen used in the assay.

A third objective is to provide improved diagnostic methods for high alcohol intake.

### The invention

We have now realized that these objectives can be achieved in case the adduct between acetaldehyde and a native protein is replaced with an adduct between acetaldehyde and a fragment of a native protein.

Accordingly the main aspect of the invention is an immunoassay method as defined below for determining body fluid IgA antibodies specific for acetaldehyde adducts. The characteristic feature is that an adduct between acetaldehyde and a fragment of a native protein is used as the antigen.

The most useful proteins are albumin and hemoglobin, i.e. the proteins that are most abundantly present in the body fluid contemplated. Fragments of human proteins are preferred.

By fragment is meant a part sequence of a native protein/polypeptide. The fragment may be obtained either by cleavage of a longer peptide chain followed by isolation of distinct fragment(s) or by direct recombinant production or chemical peptide synthesis of the intended fragment. With the present knowledge the most preferred fragments derive from amino terminal ends of native proteins or lysine and/or tyrosine containing surface exposed parts of native proteins. Useful amino-terminal fragments may, for instance, be found in the β-chain of hemoglobin. See for instance San George and Haberman, J. Biol. Chem. 261 (1986) 6811-6821 and Stevens et al., J. Clin. Invest. 67 (1981) 361-369. The length of the fragments may vary from 5 amino acid residues (with at least one residue providing a free amino group also present in the native protein) and upwards. Typically the fragments to be used are below 50%, such as below 10%, of the entire native polypeptide chain. At the present stage it is preferred to use fragments comprising native sequences of 5-10 amino acid residues.

The fragments may be covalently linked to analytically detectable groups or bound to carriers as described below. Spacer groups may have been inserted between the fragment and a label or a carrier in order to retain the efficient antibody binding activity of the fragment. Example of spacers are oligopeptides, preferably hydrophilic, not deriving from the native protein.

Acetaldehyde adducts may be synthesized by reacting the fragment with acetaldehyde under the appropriate conditions, normally either reducing or non-reducing. Reducing conditions results in adducts that comprise ethyl amino groups while the non-reducing conditions result in Schiff bases and optionally also in 2-methyl-imidazolidine-4-one structures. For further details see the experimental part and previous works that have involved adduct formation (Sillanaukee et al (Eur. J. Biochem. 240 (1996) 30-36); Sillanaukee et al (J. Lab. Clin. Med. 120 (1992) 42-47); Lin et al (Alcohol. Clin. Exp. Res. 17 (1993) 882-886); Thiele et al (Biochem. Pharmacol 48 (1994) 183-189); and Klassen et al (Alcohol. Clin. Exp. Res. 18 (1994) 164-171). Lin et al (Alcohol. Clin. Exp. Res. 19 (1995) 314-319).

Conventional antigen specific IgA immunoassays can be used. All of them encompass the formation of a ternary immune complex
anti-IgA --- IgA --- antigen/hapten
where IgA is the acetaldehyde specific antibody to be determined in the sample and antigen/hapten correspond to the adduct as defined above. Anti-IgA or the adduct may be used in an insoluble form or an insolubilizable form as known in the art. Insoluble forms include that the IgA or the adduct is stably linked to a carrier, such as solid phase, for instance a tube wall, a microtiter well, a monolithic or a particulate carrier etc. Monolithic and particulate carriers should preferentially be hydrophilic and/or porous. In order to enable measurement of the ternary complex either anti-IgA or the adduct may be labelled with an analytically detectable group, such as an isocope, an enzyme, an enzyme substrate, an enzyme cofactor, a fluorophor, a chromophor, a chemiluminescent group, a particle, biotin, hapten etc. Immunoassay variants utilizing labelled reactants in combination with insoluble or insolubilizable immune reactants and a separation of labelled reactant bound to the insoluble or insolubilizable immune reactants from labelled reactant not bound to the insoluble or insolubilizable immune reactants are often categorized as heterogeneous assays. Another type of immunoassays is called homogeneous and mostly comprises only soluble reactants without any need for separation. Nephelometry or biosensor techniques may be used for detecting the formation of the above-mentioned ternary complex with or without the use insoluble or insolubilizable or labelled reactants.

The conditions and order of addition of the various reactants are in principle the same as for the above-mentioned conventional assays, i.e. the sample containing IgA antibodies of the sought specificity is incubated with anti-IgA antibody and an acetaldehyde adduct of a protein fragment so that the ternary complex is formed. The amount selected of each reagents, order of addition, incubation times, temperature, pH etc are selected so as to allow for the complex to form in an amount that is related to the amount of sample IgA specific for the adduct. In the invention this means that anti-IgA and adduct normally should be in excess. Typical orders of additions are: adduct, sample, anti-IgA; anti-IgA, sample, adduct; sample, anti-IgA, adduct; adduct, anti-IgA, sample; sample, adduct, anti-IgA; and anti-IgA, adduct, sample. In case either the anti-IgA or the adduct is insoluble or insolubilizable it becomes possible to separate the immune complex formed in one step from excess reagents before adding the subsequent reagents Separation procedures may encompass washing steps in order to more effectively remove excess components used in a prior step.

Typical reactions times for each incubation step are often in che range of one or a few minutes up to several hours. Even over-night incubations may be used if required. The exact time depends on factors such as avidity/affinity of anti-IgA, pH, temperature, type of solid phase (if any). In case insoluble forms are used, for instance anti-IgA or the adduct is attached to a solid phase, very short incubation times may be accomplished in case the solid phase is in a porous form that is capable of absorbing the complete volume of the incubation mixture and have pore sizes optimized for diminishing diffusion times at the pore surfaces (see for instance US 4,708,932 (Pharmacia AB).

The temperature should be in the range of 0-40°C, with preference for 15-40°C. The pH is normally in the range of 3-11, with preference for 4-10. It is imperative that both the pH and the temperature are selected so that the conditions are non-denaturing for the reactants involved.

The body fluid sample may be derived from saliva, blood, serum or plasma, or any other body fluid that may contain adduct specific IgA antibodies

One aspect of the invention is a diagnostic method employing an immunoassay wherein an acetaldehyde adduct of a protein fragment as defined above is used. Elevated levels of IgA-titres (compared to the level for healthy normal non-alcoholic consumers) are taken as an indication of high alcohol intake.

One further aspect of the invention is a kit containing anti-IgA antibody and an acetaldehyde adduct of a fragment of a native protein as defined above. The anti-IgA antibody and the adduct may be labelled, insoluble or isolubilized as described above. They may be in soluble and/or predispensed form or in dry form, for instance lyophilized or spray dried, ready to be reconstituted in the appropriate buffer for the assay contemplated.

### Best mode

The best mode of the invention at the filing date is represented in the experimental part.

### EXPERIMENTAL PART

### Example 1 A: Synthesis of the acetaldehyde of a protein fragment (the five first amino acids of the N-terminal end of human hemoglobin plus a carboxy terminating cysteine (VHLTPEC).

The peptide was synthesized as earlier described (Sillanaukee et al., Eur. J. Biochem. 249 (1996) 30-46). The peptide was dissolved to 0.5 mg/ml in PBS pH 7.4. To 10 ml of the solution was then added 5 ml of 2.5 mM acetaldehyde dissolved in PBS and 5 ml of 250 mM sodium cyanoborohydride (NaCNBH₃) in PBS. The mixture was then allowed to incubate at ambient temperature for 48 hours in a tightly sealed container. The peptide solution can be stored at -20°C to +4°C in 0.5 ml aliquots.

### Example 1B: Synthesis of the adduct with bovine serum albumin.

The adduct was prepared as described in Worrall et al., Alcoholism: Clin. Exp. Res. 20 (1996) 836-840.

### Example 1C: Synthesis of the adduct with human hemoglobin

The adduct was prepared as described for bovine serum albumin in Worrall et al., Eur. J. Clin. Invest. 21 (1991) 90-95.

### Example 2: Assay of IgA reactive antibodies in serum from patients and controls.

Patients: Sera was collected from non-alcoholic women and men with a well documented drinking history attending a voluntary health screening and from alcoholics on admission to a detoxification centre in the city of Tampere, Finland. The sera had been previously analysed in ELISA using acetaldehyde modified ESA as the coating antigen (Worrall et al., Alcoholism: Clin. Exp. Res. 20 (1996) 836-840). Based on the ELISA results 10 samples with a high IgA adduct specific c reactivity (ASR) were chosen as positive controls and 10 samples with low IgA adduct specific reactivity (ASR) were chosen as negative controls.

Results: Figure 1 represents the result of Example 2a (peptide adduct). Figure 2 represents the results of Example 2B (hemoglobin adduct, Figure 3 represents the results of Example 3B (albumin adduct).

### Example 2A. Assay protocol - peptide adduct:

Preparation of microtiter wells: Aliquots of 100 µl of modified and unmodified peptide (50 µg/ml), respectively, in sodium bicarbonate buffer pH 9.6 were added to the wells of a microtiter plate and allowed to incubate at room temperature for 1 hour. The wells were then washed 4x with PBS-Tween followed by addition to each well of 200 µl of blocking solution (1 % BSA in PBS, pH 7.4) and incubation for one hour at room temperature, whereafter the wells were washed 4x with PBS-Tween.

Assay: 100 µl of patient serum diluted 1:20 (10 % chicken serum - PBS) were added to each well and incubated for 1 hour at room temperature, whereafter the wells were washed 4x with PBS-Tween. To each well was added 100 µl of β-galactosidase-Fab anti-IgA antibody conjugate (2.64 µg/ml) followed by incubation for 1 hour at 37°C and washing 4x with PBS-Tween. 100 µl of o-nitrophenyl-β-galactoside were then added to each well and allowed to incubate for 1 hour at 37°C. 100 µl stop solution was the added and the absorbance read (405 nm).

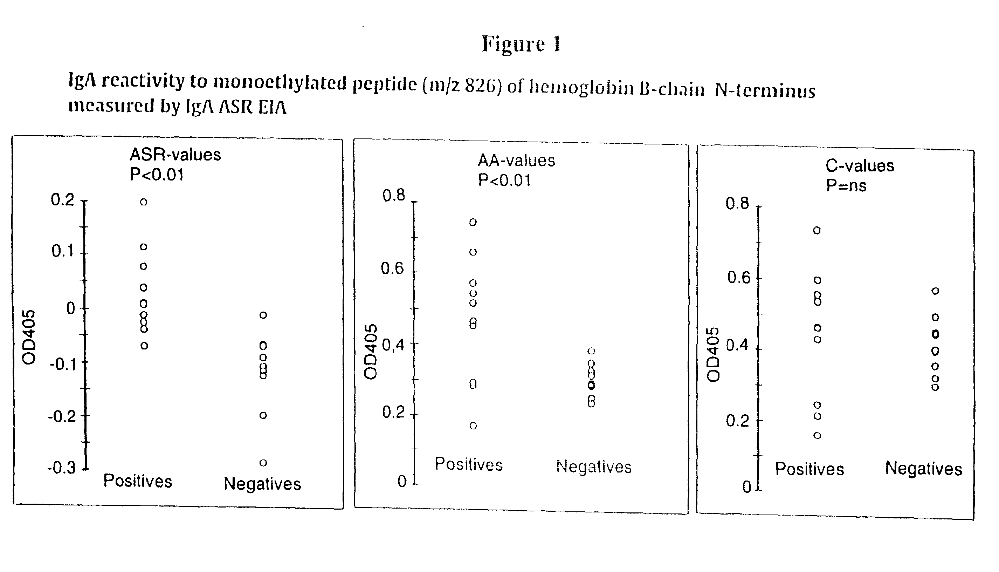

The results are shown in Figures 1a-c. Figure 1a shows the results with unmodified peptide, figure 1b the result with modified peptide (AA) and figure 1c the result if the absorbance for unmodified peptide is subtracted from the absorbance for modified peptide (adauct specific reactivity = ASR) .

### Example 2B. Assay protocol - hemoglobin adduct.

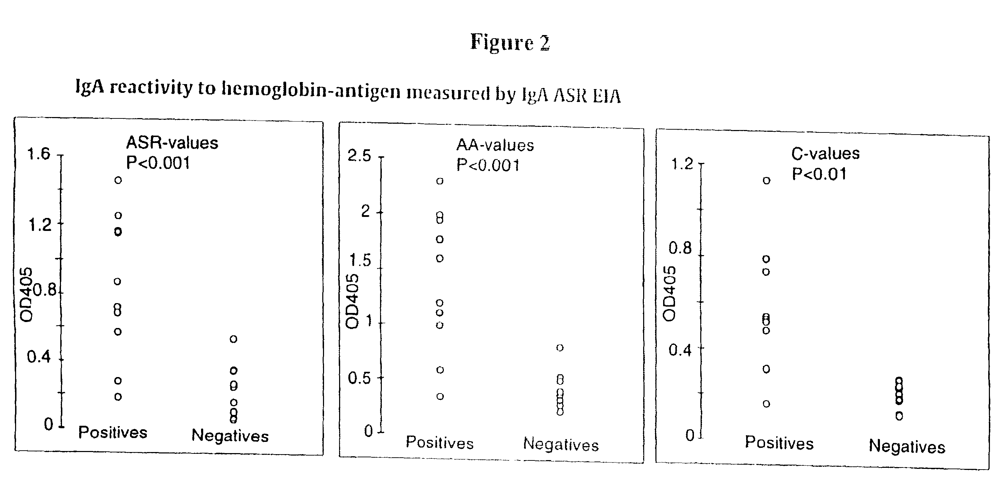

The protocol is the same as for the peptide adduct except; that the peptid- antigen is replaced with the corresponding adduct of hemoglobin (Example 1C). The results are presented in Figure 2a-c. Each of the figures 2a-c has correspondence in respective figure 1a-c.

### Example 2C. Assay protocol - albumin adduct.

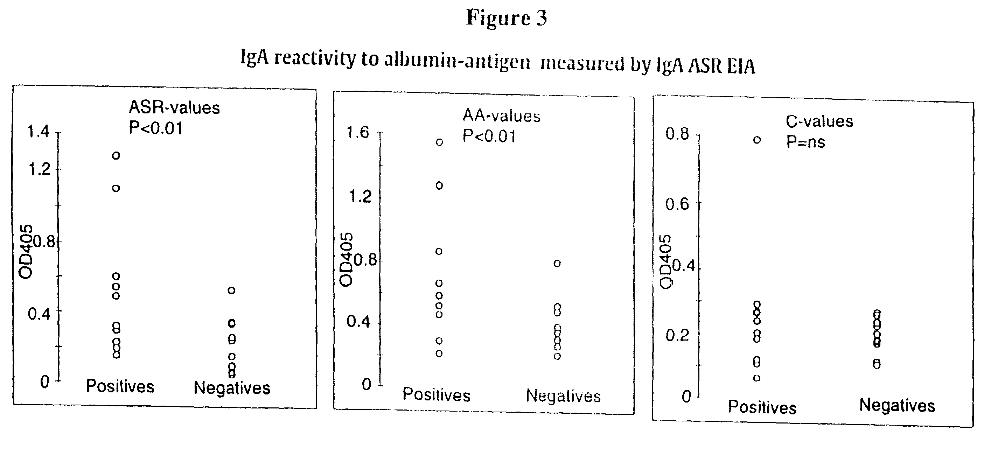

The protocol is the same as for the peptide adduct except that the peptide antigen is replaced with the corresponding adduct of bovine serum albumin (Example 1B). The results are presented in Figure 3a-c. Each of the figures 3a-c has correspondence in respective figure 1a-c.

### Conclusions

The results indicates that adducts of protein fragments are superior to protein adducts. Firstly, the unspecific binding to carrier protein, which can be seen with the hemoglobin-acetaldehyde adduct, could be avoided by using a peptide adduct. Secondly, the peptide adduct showed better sensitivity and specificity as compared to the BSA-adduct. Thirdly, the fact that alcoholics and controls did not show any significant difference in reactivity to unmodified peptide might enable the use of AA-values alone.

## Claims

1. An immunoassay method for detecting an acetaldehyde protein adduct specific IgA antibody in a body fluid sample employing an acetaldehyde adduct as the antigen, **characterized in that** said acetaldehyde adduct is an adduct of acetaldehyde and a fragment of a native protein.

2. The method according to claim 1, wherein said native protein is hemoglobin.

3. The method according to either of claims 1 and 2 wherein said fragment is N-terminal in said native protein.

4. The method according to any one of claims 1 to 3, wherein said immunoassay involves formation of the ternary complex
anti-IgA -- IgA -- adduct
where IgA stands for adduct specific IgA antibody in the sample and anti-IgA and adduct are added reagents.

5. The method according to claim 4, wherein said adduct is bound to a solid phase and said anti-IgA is labelled.

6. A method for diagnosing high alcohol intake, **characterised in that** the method of any one of claims 1-5 is used and that an increased level of acetaldehyde protein adduct specific IgA antibody in a sample from an individual, compared to healthy normal alcohol consumers, is taken as an indication of high alcohol intake.

7. A test kit containing an anti-IgA antibody and an adduct of acetaldehyde and a fragment of a native protein.

8. A test kit according to claim 7 **characterised in that** the said fragment is N-terminal in said native protein.

9. A test kit according to either of claims 7 and 8 wherein said native protein is albumin or hemoglobin.

## Patentansprüche

1. Immunoassay-Verfahren zum Nachweis eines für ein Acetaldehyd-Protein-Addukt spezifischen IgA-Antikörpers in einer Körperflüssigkeitsprobe unter Einsatz eines Acetaldehyd-Addukts als Antigen, **dadurch gekennzeichnet, dass** es sich bei dem Acetaldehyd-Addukt um ein Addukt von Acetaldehyd und eines Fragments eines nativen Proteins handelt.

2. Verfahren nach Anspruch 1, wobei es sich bei dem nativen Protein um Hämoglobin handelt.

3. Verfahren nach Anspruch 1 oder 2, wobei das Fragment im nativen Protein N-terminal ist.

4. Verfahren nach einem der Ansprüche 1 bis 3, wobei der Immunoassay die Bildung des ternären Komplexes
anti-IgA - IgA - Addukt
beinhaltet, worin IgA für den Addukt-spezifischen IgA-Antikörper in der Probe steht und es sich bei anti-IgA und dem Addukt um zugesetzte Reagenzien handelt.

5. Verfahren nach Anspruch 4, wobei das Addukt an eine Festphase gebunden und der anti-IgA markiert ist.

6. Verfahren zur Diagnose hohen Alkoholgenusses, **dadurch gekennzeichnet, dass** man das Verfahren nach einem der Ansprüche 1 bis 5 anwendet und man eine erhöhte Konzentration des für das Acetaldehyd-Protein-Addukt spezifischen IgA-Antikörpers in einer Probe einer Person im Vergleich zu gesunden Normal-Alkoholkonsumenten als Anzeichen hohen Alkoholgenusses nimmt.

7. Testkit, enthaltend einen anti-IgA-Antikörper und ein Addukt von Acetaldehyd und eines Fragments eines nativen Proteins.

8. Testkit nach Anspruch 7, **dadurch gekennzeichnet, dass** das Fragment im nativen Protein N-terminal ist.

9. Testkit nach Anspruch 7 oder 8, wobei es sich bei dem nativen Protein um Albumin oder Hämoglobin handelt.

## Revendications

1. Procédé de dosage immunologique pour détecter un anticorps IgA spécifique d'un adduit protéine acétaldéhyde dans un échantillon de liquide corporel employant un adduit acétaldéhyde en tant qu'antigène, **caractérisé en ce que** ledit adduit acétaldéhyde est un adduit d'acétaldéhyde et d'un fragment d'une protéine native.

2. Procédé selon la revendication 1, dans lequel ladite protéine native est l'hémoglobine.

3. Procédé selon l'une des revendications 1 et 2, dans lequel ledit fragment est la partie N-terminale de ladite protéine native.

4. Procédé selon l'une quelconque des revendications 1 à 3, dans lequel ledit dosage immunologique implique la formation du complexe ternaire
anti-lgA - - IgA -- adduit
dans lequel IgA représente l'anticorps IgA spécifique de l'adduit dans l'échantillon et anti-lgA et adduit sont des réactifs ajoutés.

5. Procédé selon la revendication 4, dans lequel ledit adduit est lié à une phase solide et ledit anti-IgA est marqué.

6. Procédé pour diagnostiquer une prise élevée d'alcool, **caractérisé en ce que** le procédé selon l'une quelconque des revendications 1 à 5 est utilisé, et **en ce qu'**un niveau accru d'anticorps IgA spécifique de l'adduit protéine acétaldéhyde dans un échantillon chez un individu, comparé à des consommateurs normaux d'alcool en bonne santé, est pris comme une indication de prise élevée d'alcool.

7. Kit de test contenant un anticorps anti-IgA et un adduit d'acétaldéhyde et d'un fragment d'une protéine native.

8. Kit de test selon la revendication 7, **caractérisé en ce que** ledit fragment est la partie N-terminale de ladite protéine native.

9. Kit de test selon l'une des revendications 7 et 8, dans lequel ladite protéine native est l'albumine ou l'hémoglobine.
